# EUROPEAN PATENT APPLICATION

(11) **EP 4 242 312 A1**
(43) Date of publication of application: **13.09.2023**
(21) Application number: 21889628.0
(22) Date of filing: 05.11.2021
(51) Int. Cl.: C12N 15/70, C12N 9/10, C12N 9/04, C12N 9/16, C12N 9/88, C12N 9/02, C12N 9/12

(54) **MICROORGANISMS HAVING CAPABILITY OF PRODUCING 3-HYDROXYPROPIONIC ACID FROM GLUCOSE AND USES THEREOF**

(30) Priority: 05.11.2020 KR 20200147145; 09.11.2020 KR 20200148912
(71) Applicant: Lg Chem, Ltd., Seoul 07336 (KR)
(72) Inventor: JO, Jung Hyun, Daejeon 34122 (KR); SUNG, Min Kyung, Daejeon 34122 (KR); KIM, Jae Hyung, Daejeon 34122 (KR)
(74) Representative: Goddar, Heinz J.
(86) International application number: PCT/KR2021/016058
(87) International publication number: WO 2022/098163

(57) **Abstract**

Provided is a microorganism having the capability of producing 3-hydroxypropionic acid from glucose, and a method for producing 3-hydroxypropionic acid from glucose by using the microorganism. The microorganism can include a mutation adapted to utilize intracellularly introduced glucose in 3HP production rather than cell growth, and thus increases in 3HP productivity (3HP production capacity) can be achieved. Also provided is a method that can increase 3HP production yield by controlling the time of adding an inducer and/or kinds of an alkaline aqueous solution during culturing.

## Description

### [TECHNICAL FIELD]

### Cross-reference to related applications

The present application claims the priority based on Korean Patent Application No. 10-2020-0147145 filed on November 05, 2020 and Korean Patent Application No. 10-2020-0148912 filed on November 09, 2020, and all the contents disclosed in the documents of the corresponding Korean Patent Applications are incorporated by reference as a part of the present description.

The present description relates to a microorganism having production capability of 3-hydroxypropionic acid from glucose, and a method for producing 3-hydroxypropionic acid from glucose using the microorganism.

### [BACKGROUND ART]

3-Hydroxypropionic acid (3-HP) is an important synthetic intermediate used in various chemical processes, and key compounds synthesized using 3-HP as a precursor include 1,3-propandiol, acrylic acid, methyl acrylate, acrylamide, ethyl 3-HP, malonic acid, propiolactone and acrylonitrile, and the like. In addition, 3-HP has excellent economic value to the extent that it has been selected as an important high value-added compound that can be converted from biomass by US DOE (Department of Energy) in 2004.

3-HP can be produced through a chemical process using ethylene cyanohydrin, beta-iodopropionic acid, beta-bromopropionic acid, beta-chloropropionic acid, beta-propiolactone, acrylic acid, and the like as an intermediate. However, there are problems in that most of these chemicals are toxic and carcinogenic and consume a large amount of energy under high temperature and pressure conditions, and discharge a large amount of pollutants.

3-HP also can be produced through a biological process from glycerol. For example, 3-HP can be produced through microbial fermentation. Until now, a 3-HP production process using E. coli or Klebsiella pneumoniae, or the like has been studied, but it has not yet reached a commercial level in terms of productivity, yield and economic feasibility of the medium. Most of the studies to date are those that synthesize 3-HP using glycerol as a substrate, and there have been few studies on a technology of biosynthesizing 3-HP from glucose other than glycerol, and there is a problem in that the yield of 3-HP production is lowered as glucose is also used for cell growth. Accordingly, there is a need to study a microorganism capable of producing 3-HP from glucose and a method for producing 3-HP using the same.

### [PRIOR ART]

Korean Granted Patent No. 10-1689451

### [DISCLOSURE]

### [TECHNICAL PROBLEM]

Provided is a microorganism having production capability of 3-hydroxypropionic acid from glucose.

The microorganism can have the following characteristics:
(1) enhancement of activity of adenosyltransferase; and
(2) enhancement of activity of glycerol-3-phosphate dehydrogenase (GPD), glycerol-3-phosphate phosphatase (GPP), or both of them.

The microorganism can further have one or more characteristics selected from the following:
(3) enhancement of activity of one or more enzymes selected from the group consisting of glycerol dehydratase, glycerol dehydratase reactivase, and aldehyde dehydrogenase;
(4) enhancement of activity of galactose permease, glucokinase, or both of them; and
(5) weakening or inactivation of activity of a glucose-specific transporter of the phosphotransferase system.

The microorganism can be a microorganism belonging to the genus Escherichia.

The microorganism can increase production capability of 3-hydroxypropionic acid from glucose and/or reduce production of byproducts (for example, glycerol, etc.), compared to a homogeneous microorganism not having one or more characteristics selected from the characteristics (1) to (5).

Also provided is a composition for producing 3-hydroxypropionic acid, the composition comprising the microorganism.

Also provided is a method for production of 3-hydroxypropionic acid, comprising culturing the microorganism in a medium containing glucose.

Also provided is a culture obtained by culturing the microorganism in a medium containing glucose. The culture can comprise 3-hydroxypropionic acid in an amount of 60g or more per 1L and not comprise glycerol or comprise glycerol in an amount of 20g or less per 1L.

### [TECHNICAL SOLUTION]

The present application provides a technology related to a microorganism with increased production capability of 3-hydroxypropionic acid (for example, 3-HP production and 3-HP production yield) from glucose, and a method for producing 3-hydroxypropionic acid from glucose using the microorganism.

Hereinafter, the present application will be described in more detail.

### Microorganism having production capability of 3-hydroxypropionic acid (3-HP) from glucose

One example of the present application provides a microorganism having production capability of 3-hydroxypropionic acid from glucose.

The microorganism can have the following characteristics:
(1) enhancement of activity of adenosyltransferase; and
(2) enhancement of activity of glycerol-3-phosphate dehydrogenase (glycerol-3-phosphate dehydrogenase; GPD), glycerol-3-phosphate phosphatase (glycerol-3-phosphate phosphatase; GPP), or both GDP and GPP.

The microorganism can further have one or more characteristics selected from the following:
(3) enhancement of activity of one or more enzymes selected from the group consisting of glycerol dehydratase, glycerol dehydratase reactivase, and aldehyde dehydrogenase;
(4) enhancement of activity of galactose permease, glucokinase, or both of them; and
(5) weakening or inactivation of activity of a glucose-specific transporter of the phosphotransferase system.

In one example, the microorganism can comprise all the characteristics of (1) to (5).

The microorganism can be an Escherichia sp. microorganism.

The microorganism can increase production capability of 3-hydroxypropionic acid from glucose and/or reduce production of by-products (for example, glycerol, acetate, PDO, etc.), compared to a homogeneous microorganism which does not have one or more characteristics selected from the characteristics (1) to (5).

In the present application, 'enhancement, weakening and/or inactivation of activity' can mean that the activity is enhanced, weakened, and/or inactivated, compared to a parent strain (host microorganism). The parent strain may mean a homogeneous strain to the microorganism, which is a microorganism in which a mutation to have one or more characteristics selected from (1) to (5) is not introduced or is before introduction.

In the present application, "adenosyltransferase (corrinoid adenosyltransferase)" can mean an enzyme causing delivery of a deoxyadenosyl residue of reduced corrinoid in ATP, and any enzyme having the activity can be included in the scope of the present application, and it can be derived from various kinds of prokaryotes or eukaryotes. For example, the adenosyltransferase can be an enzyme belonging to EC 2.5.1.17, and can be derived from *Escherichia coli, Pseudomonas denitrificans, Salmonella enterica, Salmonella typhimurium, Shewanella oneidensis, Ralstonia solanacearum, Pseudomonas denitrificans,* or *Mycobacterium bovis,* and the like, and the gene encoding this can include *btuR* (GenBank Accession Nos. M21528.1), *cobA* (GenBank Accession Nos. L08890.1), or *cobO* (GenBank Accession Nos. M62866.1), or the like.

In the present application, "glycerol-3-phosphate dehydrogenase (GPD)" can mean a polypeptide having enzymatic activity which catalyzes conversion from dihydroxyacetone phosphate (DHAP) to glycerol-3-phosphate (3GP). Glycerol-3-phosphate dehydrogenase can be NADH-, NADPH-, or FAD-dependent. Glycerol-3-phosphate dehydrogenase, regardless of its origin, can be included in the scope of the present application, as long as it has enzymatic activity that catalyzes conversion from DHAP to G3P, and for example, the gene encoding this may be GPD1 and/or GPD2, and it may be derived from *Saccharomyces cerevisiae,* and it may be GPD1 (Genbank Accession no. CAA98582.1), GPD2 (Genbank Accession no. NM_001183314.1), or combination thereof.

In the present application, "glycerol-3-phosphate phosphatase (GPP)" refers to a polypeptide having enzymatic activity which catalysts conversion from glycerol-3-phosphate (G3P) to glycerol. Glycerol-3-phosphate phosphatase, regardless of its origin, can be included in the scope of the present application, as long as it has enzymatic activity that catalyzes conversion from G3P to glycerol, and for example, the gene encoding this can be GPP1 and/or GPP2, and it can be derived from *Saccharomyces cerevisiae,* and it may have GPP1 (Genbank Accession no. KZV10562.1; encoding gene: Genbank Accession no. NM_001179403.1), GPP2 (Genbank Accession no. NP_010984.3; encoding gene: Genbank Accession no. NM_001178953.3).

In the present application, "glycerol dehydratase " or "diol dehydratase" can mean a polypeptide having enzymatic activity which catalysts conversion from glycerol to 3-hydroxypropionaldehyde (3-HPA). Glycerol dehydratase or diol dehydratase can be coenzyme B12 dependent or non-dependent. The enzyme can be derived from *Klebsiella pneumoniae, Citrobacter freundii, Clostridium pasteurianum, Salmonella typhimurium, Klebsiella oxytoca,* and/or *Clostridium butyricum,* and the like, but is not limited thereto. The enzyme, regardless of its origin, may be included in the scope of the present application, as long as it has enzymatic activity that catalyzes conversion from glycerol to 3-hydroxypropionaldehyde (3-HPA), and for example, the gene encoding this may be dhaB. In one example, the dhaB may be derived from Klebsiella pneumoniae (EMBL Accession Nos. U30903.1(dhaB1), Genbank Accession no. CDO12380.1 (dhaB), etc.).

In the present application, "glycerol dehydratase reactivase" may mean an enzyme which acts to maintain catalytic activity by reactivating what is irreversibly inactivated during the action of glycerol dehydratase. Any commonly known glycerol dehydratase reactivase may be used, and for example, it may be derived from Klebsiella sp. (for example, Klebsiella pneumoniae), Citrobacter sp., Lactobacillus sp., Salmonella sp. strain, and the like, but is not limited thereto. Glycerol dehydratase reactivase may be DhaFG, GdrAB, DdrAB, and the like, and for example, the gene encoding this may be dhaFG, gdrAB, and/or ddrAB.

In the present application, "aldehyde dehydrogenase" is an enzyme which converts aldehyde into carboxylic acid, and may mean a polypeptide having enzymatic activity which catalysts conversion from 3-hydroxypropionaldehyde (3-HPA) to 3-hydroxypropionic acid (3-HP). Any commonly known aldehyde dehydrogenase can be used, and for example, it can be derived from Escherichia sp. (for example, Escherichia sp. (for example, E. coli)), and the like, but is not limited thereto. Any enzyme having enzymatic activity which catalyzes conversion of 3-hydroxypropionaldehyde (3-HPA) to 3-hydroxypropionic acid (3-HP) can be includes in the scope of the present application, and for example, the gene encoding this can be aldH and/or puuC, and the like.

In the present application, "galactose permease (GalP)" can mean a protein which acts to transfer various kinds of saccharides including galactose and/or glucose into cells (Venter, Henrietta et al., Biochemical Journal (2002)363:243-252). The galactose permease (GalP) is a symporter which can act as glucose permease and has 12 trans-membrane loops, and can have the ability to transport glucose across cell membranes. The galactose permease (GalP) can transfer glucose in the pts-strain, and can act to compensate for loss of glucose uptake mediated by phosphotransferase system (PTS). In one example, the galactose permease can be galactose permease derived from E. coli (for example, W3110 strain), and it can be encoded by a GalP gene.

In the present application, "glucokinase" can mean an enzyme which promotes phosphorylation of glucose to glucose 6-phosphate. For example, the glucokinase can be glucokinase derived from E. coli (for example, W3110 strain), and can be encoded by a glk gene. In one example, it can phosphorylate transported glucose in the environment that the phosphotransferase system (PTS) consuming PEP (phosphoenol pyruvate) is not activated, and for example, it can phosphorylate transported glucose by the galactose permease in the environment lacking PTS.

In the present application, "glucose-specific transporter of the phosphotransferase system" can mean a protein capable of transporting glucose, in the phosphotransferase system, and for example, it can be EIICB^{Glc} (Enzyme IICB^{Glc}). In one example, the glucose transporter of the phosphotransferase system can be derived from E. coli (for example, W3110 strain), and can be encoded by ptsG. The "phosphotransferase system (PTS)" is a system to transport glucose and other saccharides from bacteria into cells, and it can use phosphate and PEP (phosphoenol pyruvate) as an energy source to transport saccharides and simultaneously phosphorylate them.

The microorganism according to one example can intake and use glucose using a pathway of glucose intake not using PEP, as the activity of the glucose transporter of the phosphotransferase system is weakened and/or inactivated and the activity of galactose permease and glucokinase is enhanced. The microorganism according to one example can transport glucose into cells and convert transported glucose into a glucose-6-phosphate form, as the activity of galactose permease and glucokinase is enhanced, even though the activity of the glucose transporter of the phosphotransferase system is weakened and/or inactivated, and this process may not require PEP and/or may not depend on PEP. In one example, the microorganism can intake and/or use glucose by a saccharide introduction system.

The microorganism provided in the present invention can have:
(1) enhancement of activity of adenosyltransferase,
(2) enhancement of activity of glycerol-3-phosphate dehydrogenase (GPD), glycerol-3-phosphate phosphatase (GPP), or both of them,
(3) enhancement of activity of one or more selected from the group consisting of glycerol dehydratase, glycerol dehydratase reactivase, and aldehyde dehydrogenase,
(4) enhancement of activity of galactose permease, glucokinase, or both of them, and
(5) weakened or inactivation of activity of a glucose-specific transporter of the phosphotransferase system.

More specifically, the microorganism can have enhanced activity of adenosyltransferase. In one example, the enhancement of the activity of adenosyltransferase can be due to an increase in the expression of the btuR gene. The increase of the expression can be caused by introduction of a homogeneous or heterogeneous btuR gene. Such enhancement of the activity of adenosyltransferase can reduce intracellular accumulation of glycerol obtained from glucose metabolism and promote conversion of glycerol into 3-HP, thereby improving the production yield of 3-HP from glucose.

In addition, the microorganism can have enhanced activity of glycerol-3-phosphate dehydrogenase (GPD) and/or glycerol-3-phosphate phosphatase (GPP), and it can be caused by overexpression of a glycerol-3-phosphate dehydrogenase encoding gene and/or glycerol-3-phosphate phosphatase encoding gene. In one example, the microorganism can further comprise a gene encoding homogeneous or heterogeneous GPD and a gene encoding GPP, and for example, it can additionally comprise a gene encoding GPD and/or a gene encoding GPP which are operably linked to an inducible promoter, respectively. The inducible promoter can induce expression of a gene linked to the promoter by addition (treatment) of an inducer, and can control the transcriptional timing of the gene encoding GPD and gene encoding GPP, which are foreign genes, using the inducible promoter. According to one example, the inducible promoter can be BAD, LTTR, BAD, Trc, and/or Tac, and when the inducible promoter is BAD, the inducer can be L-arabinose, and when the inducible promoter is LTTR, the inducer can be 3-HP. According to one specific example, the microorganism can further comprise the GPD gene and GPP gene operably linked to the LTTR promoter.

The BAD, LTTR, and/or TAC promoter can comprise the nucleic acid sequence described in Table 1 below or consist of the nucleic acid sequence described in Table 1.

**[Table 1]**

| Name | Sequence (5'->3') | SEQ ID NO: |
|---|---|---|
| TAC | ttgacaattaatcatcggctcgtataatgtgt | SEQ ID NO: 24 |
| BAD | AAAGCCATGACAAAAACGCGTAACAAAAGTGTCTATAA TCACGGCAGAAAAGTCCACATTGATTATTTGCACGGC GTCACACTTTGCTATGCCATAGCATTTTTATCCATAAG ATTAGCGGATCCTACCTGACGCTTTTTATCGCAACTCT CTACTGTTTCTCCAT | SEQ ID NO: 25 |
| LTTR | GTCAGCCTCAGCGCACCTCGAATGTGCAAAAACGCAG ACCATACTTGCACATCACCGCATTGAGTACATCAAAAA TGCACTGTTAGGATCGATCCAGACAACAAAAAAGCCA CA | SEQ ID NO: 5 |

In one example, the gene encoding glycerol-3-phosphate dehydrogenase in which the inducible promoter is introduced, and the gene encoding glycerol-3-phosphate phosphatase in which the inducible promoter is introduced, can be cloned in a vector, respectively, to be introduced into a cell. Otherwise, the two genes can be cloned in one vector to be introduced into a cell. Herein, the vector refers to a gene construct comprising an essential regulatory element operably linked to express a gene insert encoding a target protein in a cell of a subject, and various types of vectors such as a plasmid, a virus vector, bacteriophage vector, a cosmid vector, and the like can be used.

In addition, the microorganism can have enhanced activity of one or more enzymes selected from the group consisting of glycerol dehydratase, glycerol dehydratase reactivase, and aldehyde dehydrogenase. In one example, the microorganism can further comprise one or more selected from the group consisting of dhaB gene encoding glycerol dehydratase, gdrAB gene encoding glycerol dehydratase reactivase and aldH gene encoding aldehyde dehydrogenase, or for example, all three of them, and thereby, the microorganism according to one example can have enhanced activity of glycerol dehydratase, glycerol dehydratase reactivase, and/or arabinose transport protein as the dhaB, gdrAB, and/or aldH genes are overexpressed. The microorganism according to one example can additionally comprise a constitutive expression promoter and dhaB, gdrAB, and/or aldH operably linked thereto. The constitutive expression promoter can induce expression of the operably linked gene without a separate inducer. The constitutive expression promoter can be J23100, J23101, J23102, J23103, J23104, J23105, J23106, J23107, and/or J23109, but is not limited thereto, and can be selected and used without limitation, if necessary, within a range of purpose of producing 3-HP by those skilled in the art. According to one specific example, the microorganism can further comprise the dhaB gene and gdrB gene operably linked to J23101 promoter and the aldH gene operably linked to J23100.

In one example, the gene encoding glycerol dehydratase in which the constitutive expression promoter is introduced, the gene encoding glycerol dehydratase reactivase in which the constitutive expression promoter is introduced, and the gene encoding arabinose transport protein in which the constitutive expression promoter is introduced can be cloned in a vector, respectively, to be introduced into a cell. Otherwise, the three genes can be cloned in one vector to be introduced into a cell.

In addition, the microorganism can have enhanced activity of galactose permease and/or glucokinase. In one example, the enhancement of the activity of galactose permease can be caused by an increase of expression of galP gene, and the enhancement of the activity of glucokinase can be caused by an increase of expression of glk gene. The increase of expression can be due to introduction of homogeneous or heterogenous galP gene and/or glk gene.

Furthermore, the microorganism can have weakened or inactivated activity of the glucose-specific transporter of the phosphotransferase system. In one example, in the microorganism, the weakening and/or inactivation of activity of the glucose-specific transporter of the phosphotransferase system can be due to deletion of all and/or part of the ptsG gene inherent in the microorganism.

In the present description, the term "protein (or polypeptide)" can mean a protein which has identity of 80%, 85%, 90%, 95%, 96%, 97%, 98% or 99% or more to the amino acid sequence of the disclosed protein, and has an enzymatic activity and/or effect substantially equal to the protein of the amino acid sequence (that is, an effect of giving or enhancing production capability of 3-hydroxypropionic acid from glucose). In addition, the term "gene (or polynucleotide)" can mean a nucleic acid molecule encoding a protein which has identity of 80%, 85%, 90%, 95%, 96%, 97%, 98% or 99% or more to the nucleic acid sequence of the disclosed gene, and has an enzymatic activity and/or effect substantially equal to the protein encoded by the nucleic acid sequence (that is, an effect of giving or enhancing production capability of 3-hydroxypropionic acid from glucose).

Herein, "% identity" of sequences refers to a level to which bases are identical when two or more polynucleotides are aligned so that they are most identical to each other and then the sequences are compared. Sequence identity percent can be calculated, for example, by comparing two optimally aligned sequences throughout the comparison region and determining the number of positions where the same amino acid or nucleic acid appears in both sequences to obtain the number of matched positions, and dividing the number of matched positions by the total number of positions within the comparison range (i.e., range size), and multiplying the result by 100 to obtain the percent of sequence identity. The percent of sequence identity can be determined using a known sequence comparison program, and the program can include BLASTN (NCBI), CLC Main Workbench (CLC bio), MegAlign^{™} (DNASTAR Inc), and the like, as one example.

Herein, "enhancement of activity" or "enhanced activity" includes that the activity of a protein itself is newly introduced or increased to induce an effect beyond its original function, and as well, it can mean a state in which the activity of the microorganism after manipulation is increased, compared to the activity of the microorganism before manipulation such as an increase in endogenous gene activity, amplification of an intrinsic gene from internal or external factors, deletion of an inhibition regulator of the gene expression, an increase in gene copy number, gene introduction from outside, modification of an expression regulatory sequence, and/or replacement or modification of a promoter, and an increase in enzymatic activity by an intragenic mutation.

In one example, the enhancement of activity of the protein (enzyme) can be achieved by application of various methods well known in the art. As the method for enhancing or increasing the activity of the enzyme, various methods well known in the art can be applicable. For example, but not limited thereto, a method for inserting a gene (polynucleotide) comprising a base sequence encoding the corresponding enzymes (or proteins) into chromosome additionally, a method for increasing the copy number of a base sequence encoding enzymes (or proteins) by a method for introducing the gene (polynucleotide) into a vector system, a method for replacing a promoter capable of expressing a gene (polynucleotide) with a strong promoter, and a method for introducing a mutation into a promoter can be included, and there is a method for mutating into an enzyme (or protein) with strong activity by genetic mutation, and the like. The method for inserting the gene (polynucleotide) into host cell genome (chromosome) can be performed by appropriately selecting a known method by those skilled in the art, and for example, it can be performed using an RNA-guided endonuclease system (for example, one or more selected from the group consisting of (a) RNA-guided endonuclease (e.g., Cas9 protein, etc.), its encoding gene, or a vector comprising the gene; and (b) guided RNA (e.g., single guide RNA (sgRNA), etc.), its encoding DNA, or a mixture comprising a vector comprising the DNA (for example, a mixture of RNA-guided endonuclease protein and guided RNA, etc.), a complex (for example, ribonucleic acid fusion protein (RNP), a recombinant vector (for example, a vector comprising RNA-guided endonuclease encoding gene and guided RNA encoding DNA together, etc.), and the like), but is not limited thereto.

The microorganism according to one example can have enhanced activity of the protein (or enzyme) encoded by the gene by additionally comprising a constitutive expression promoter and a gene operably linked thereto. The constitutive expression promoter can induce expression of the operably linked gene without a separate inducer. The constitutive expression promoter can be J23-based (for example, J23100, J23101, J23102, J23103, J23104, J23105, J23106, J23107, and/or J23109), but is not limited thereto, and it can be used by selecting without limitation, if necessary, within a range of purpose for producing 3-HP by those skilled in the art. In one example, the enhancement of activity of protein (or enzyme) can mutate a promoter into a strong promoter compared to a native promoter through mutation or replacement. An improved promoter or heterogeneous promoter having a base replacement mutation can be linked instead of the promoter of the intrinsic enzyme.

Herein, "promoter" refers to an untranslated nucleic acid sequence upstream of an encoding region which comprises a binding site for polymerase and has the activity of initiating transcription into mRNA of a promoter downstream gene, that is, DNA region which polymerase binds to and induces transcription of the gene, and can be positioned at the 5' site of the mRNA transcription initiation site.

Herein, "vector" refers to any medium for cloning and/or transferring of a base into a host cell. The vector can be a replicon capable of binding other RNA fragment to bring about replication of the bound fragment. "Replicon" refers to any genetic unit (for example, plasmid, phage, cosmid, chromosome, virus) that functions as a self-unit of DNA replication *in vivo,* i.e., capable of replicating by its own regulation. In the present invention, the vector is not particularly limited as long as it is capable of replication in a host, and any vector known in the art can be used. The vector used for construction of the recombinant vector can be natural or recombinant plasmid, cosmid, virus and bacteriophage. For example, as the phage vector or cosmid vector, pWE15, M13, λEMBL3, λEMBL4, λFIXII, λDASHII, λZAPII, λgt10, λgt11, Charon4A, and Charon21A, and the like can be used, and as the plasmid vector, pDZ vector, pBR-based, pUC-based, pBluescriptll-based, pGEM-based, pTZ0-based, pCL-based and pET-based, and the like can be used. The usable vector is not particularly limited, and a known expression vector can be used, but is not limited thereto. In one example, the vector can be one such that the gene transferred by being inserted into the vector is irreversibly fused into the genome of the host cell so that the gene expression in the cell is stably maintained for a long time. Such vector comprises transcriptional and translational expression regulatory sequences which allow the gene of interest to be expressed in the selected host. As the expression regulatory sequence, any operator sequence for regulating transcription and/or a sequence regulating termination of transcription and translation. An initiation codon and a termination codon are generally considered a part of a nucleic acid sequence encoding a target protein, and when a gene construct is administered, it should be functional in a subject and should be in frame with a coding sequence. Also, in case of an expression vector capable of replication, an origin of replication can be included. In addition, an enhancer, a non-translated region at the 3' end of a target gene, a selection marker (for example, an antibiotic resistance marker), or a replication competent unit, or the like can be included appropriately. The vector can be self-replicating or integrated into host genomic DNA.

According to one example, each element in the vector should be operably linked each other, and the linking of these element sequences can be performed by ligation (linking) at convenient restriction enzyme sites, and when such a site does not exist, it can be performed using a synthetic oligonucleotide adaptor or linker according to a conventional method.

Herein, "transformation" refers to introducing a gene into a host cell so that it can be expressed in the host cell, and as long as the transformed gene can be expressed in the host cell, it can be included without limitation whether it is inserted into chromosome of the host cell or located outside the chromosome. In addition, the gene includes DNA and/or RNA encoding a target protein. As long as the gene can be introduced and expressed into a host microorganism, the introduced form is not limited. For example, the gene can be introduced into a host microorganism in the form of an expression cassette, which is a gene construct including all elements necessary for self-expression. The expression cassette can include expression regulatory elements such as a promoter, a transcription termination signal, a ribosome binding site and/or a translation termination signal, which are operably linked to the gene in general. The expression cassette can be in the form of an expression vector capable of self-replication. In addition, the gene can be introduced into a host cell in its own form and operably linked to a sequence required for expression in the host cell.

Herein, as the method for transforming, any method for introducing a gene into a cell is included without limitation, and it can be performed by selecting an appropriate standard technology according to the host cell as known in the art. For example, electroporation, calcium phosphate (CaPO₄) precipitation, calcium chloride (CaCl₂) precipitation, microinjection, retroviral infection, polyethylene glycol (PEG) method, DEAE-dextran method, cation liposome method, and/or lithium acetate-DMSO method, and the like can be used, but is not limited thereto.

Herein, "operably linked" means that a promoter sequence that initiates and mediates transcription of a target gene to be expressed and the gene sequence are functionally linked.

The microorganism according to one example can have weakened or inactivated activity of the glucose-specific transporter of the phosphotransferase system. In one example, in the microorganism, the weakening and/or inactivation of activity of the glucose-specific transporter of the phosphotransferase system can be caused by deletion of all or part of the ptsG gene.

Herein, "weakening of activity" can mean a case in which the degree of activity of the glucose-specific transporter of the phosphotransferase system in the cell is reduced compared to the parent strain, such as a wild-type strain or strain before modification. The weakening is a concept including when the activity of the enzyme itself is reduced compared to the activity of the enzyme possessed by the original microorganism due to mutation of the gene encoding the enzyme, and when the overall degree of the enzymatic activity in the cell is lower than that of the wild-type strain or strain before modification, as the expression level of the enzyme is lowered due to transcription inhibition and/or translation inhibition of the gene encoding it, and the like, and also combinations thereof. Moreover, herein, "inactivation" can mean a state in which expression of a gene encoding the glucose-specific transporter of the phosphotransferase system (for example, ptsG) is not expressed at all compared to the wild-type strain or strain before modification, or there is no activity even if it is expressed.

In one example, weakening or inactivation of activity of a protein or enzyme (for example, glucose-specific transporter of the phosphotransferase system) can be achieved by application of various methods well known in the art. For example, there are: a method for replacing a gene encoding the enzyme on the chromosome with a mutated gene so that the activity of the enzyme (or protein) is reduced including a case in which the activity of the enzyme (or protein) is completely removed; a method for introducing mutation to an expression regulatory sequence (for example, promoter) of a gene on chromosome encoding the enzyme (or protein); a method for replacing an expression regulatory sequence of a gene encoding the enzyme (or protein) with a sequence with weak activity or no activity; a method for deleting all or part of a gene on a chromosome encoding the enzyme (or protein); a method for introducing antisense oligonucleotide (for example, antisense RNA) inhibiting translation from the mRNA to the enzyme (or protein) by complementarily binding to transcriptome of the gene on the chromosome; a method for making attachment of ribosomes impossible by artificially adding a sequence complementary to the SD sequence to the front end of the SD sequence of the gene encoding the enzyme (or protein) to form a secondary structure; and/or an RTE (Reverse transcription engineering) method for adding a promoter so as to be reverse transcribed at the 3' end of an ORF (open reading frame) of the corresponding sequence, and the like, and it can be achieved by combinations thereof, but is not limited thereto.

Herein, reduction of gene expression can mean that the expression level of a gene is reduced compared to the expression level of a host cell before mutation (parent strain) and/or wild type, and can include a case that the expression is completely inhibited so that the expression is not achieved. When it is desired to reduce the expression of a gene in a microorganism, the expression can be reduced by substituting an initiation codon of gene translation, or can be genetically engineered to reduce the expression level of an existing gene. A method for modifying a sequence of expression regulation can be performed by inducing mutation in the expression regulatory sequence by deletion, insertion, non-conservative or conservative substitution, or a combination thereof to a nucleic acid sequence of the expression regulatory sequence, or can be performed by a method for replacing a promoter with a weaker promoter, and the like. The expression regulatory sequence includes a promoter, an operator sequence, a sequence encoding a ribosome binding site, and a sequence regulating termination of transcription and translation, but is not limited thereto. When a gene for reducing expression is present in the microorganism to be mutated, for example, by substituting a weak promoter with a native promoter that drives the expression of the gene or introducing mutation, the expression of the gene can be reduced. Furthermore, by deleting all or part of the existing gene, the expression of the gene can be reduced.

Herein, "microorganism" can include unicellular bacteria, and for example, it can be an Escherichia sp. microorganism including an E. coli (for example, E. coli DH5a, E. coli JM101, E. coli K12, E. coli W3110, E. coli X1776, E. coli B, and/or E. coli XL1-Blue).

In one example, the microorganism can additionally have weakened or inactivated activity of one or more enzymes selected from the group consisting of (a) to (f) below:
(a) 1,3-propanediol dehydrogenase;
(b) glycerol kinase;
(c) acetate kinase;
(d) phosphotransacetylase;
(e) lactate dehydrogenase; and
(f) glycerol dehydrogenase.

In one specific example, in the microorganism, one or more genes selected from the group consisting of (i) to (v) below can be additionally deleted:
(i) yqhD encoding 1,3-propanediol dehydrogenase, (ii) glpK encoding glycerol kinase, (iii) IdhA encoding lactate dehydrogenase, (iv) ackA-pta encoding acetate kinase-phosphotransacetylase, and/or (v) gldA gene encoding glycerol dehydrogenase.

In one specific example, in the microorganism, yqhD, glpK, IdhA, ack-pta, and gldA genes can be additionally deleted.

The microorganism according to one example can be one in which cell growth is inhibited (compared to the parent strain). The microorganism according to one example can be easily utilized for producing 3HP without using introduced glucose for cell growth than the host cell, as it can introduce glucose without requiring PEP. The microorganism according to one example can be one in which the adenosyltransferase activity is enhanced to increase conversion from glucose to 3-HP (compared to the parent strain).

The microorganism according to one example can have the ability to synthesize organic acid, and for example, it can have ability to synthesize 3-hydroxypropionic acid (or 3-HP production capability). The microorganism according to one example can be a genetically engineered strain to produce organic acid, or have organic acid production capability or enhance organic acid production capability by comprising a gene involved in organic acid production itself. The production of the organic acid includes being produced in the strain, being produced in the cell and secreted to the outside of the cell, or a combination thereof.

The 3-hydroxypropionic acid (3-HP) is an isomer of lactate (2-hydroxypropionic acid) and is used as a crosslinking agent of a polymer coating agent, a metal lubricant, an antistatic agent of fabric, and the like, and is a platform compound which can be converted into several commercially important chemical substances such as acrylic acid, 1,3-propanediol, methyl acrylate, ethyl 3-hydroxypropionic acid, malonic acid, propionic lactone, acrylonitrile or acrylamide, and the like. 3-HP can be produced through a biological process from glucose.

Herein, "having production capability of 3-hydroxypropionic acid (3-HP)" means a cell and/or a microorganism naturally having 3-HP production capability, or a microorganism with 3-HP production capability given to the parent strain that does not have 3-HP production capability. It can be used to mean when the microorganism has 3-HP production capability by introducing the mutation according to one example and/or when the microorganism has 3-HP production capability by introducing the mutation according to one example into a microorganism not having 3-HP production capability. For example, the Escherichia sp. microorganism having 3-HP production capability can mean an Escherichia sp. microorganism having improved 3-HP production capability by inserting a foreign gene related to the 3-HP production mechanism into a native microorganism itself, or enhancing or inactivating the activity of an intrinsic gene.

In one example, the microorganism can be a strain which has 3-HP production capability or is genetically engineered to have 3-HP production capability.The production of 3-HP includes being produced in the strain, being producing in the cell and excreted to the outside of the cell, or a combination thereof.

In one example, the microorganism can have a 3-HP yield when culturing in a medium comprising glucose for 24 hours (3-HP production (g)/amount of inserted glucose (g)) of 0.4 or more, 0.42 or more, 0.45 or more, 0.47 or more, 0.5 or more, 0.52 or more, 0.54 or more, 0.57 or more, 0.6 or more, 0.65 or more, 0.7 or more or 0.75 or more (the upper limit can be 1, 0.9, 0.85 or 0.8).

In addition, the microorganism can have 3-HP production when culturing in a medium comprising glucose for 24 hours to 48 hours, for example, 24 hours, 30 hours, 36 hours, 42 hours or 48 hours, of 60g/L or more, 65g/L or more, 70g/L or more, or 75g/L or more, for example, 60g/L to 200g/L, 60g/L to 150g/L, 60g/L to 100g/L, 65g/L to 200g/L, 65g/L to 150g/L, 65g/L to 100g/L, 70g/L to 200g/L, 70g/L to 150g/L, 70g/L to 100g/L, 75g/L to 200g/L, 75g/L to 150g/L, or 75g/L to 100g/L.

In addition, the microorganism can have the production of by-products (e.g., one or more selected from glycerol, acetate, PDO (1 ,3-propanediol)) of 20g/L or less, 15g/L or less, 10g/L or less, 5g/L or less, 4g/L or less, 3g/L or less, 2g/L or less, or 1.5g/L or less (the lower limit can be 0 (not producing by-products), 0.001g/L, 0.01g/L, or 0.1 g/L), when culturing in a medium comprising glucose for 24 hours to 48 hours, for example, for 24 hours, 30 hours, 36 hours, 42 hours or 48 hours.

The microorganism (or recombinant cell) can have increased (improved) 3-HP production capability than a cell before recombination, a parent strain and/or a wild-type strain. The microorganism in which the mutation according to one example is introduced can have increased 3-HP production, compared to a homogeneous non-modified microorganism. The non-modified microorganism can mean a homogeneous microorganism in which the mutation according to one example is not introduced or a microorganism before introduction. In one example, the production capability (production or production yield) of 3-HP can be increased by about 0.1% or more, about 1% or more, about 5% or more, about 10% or more, about 15% or more, about 20% or more, about 30% or more, about 50% or more, about 60% or more, about 70% or more, about 80% or more, about 90% or more, about 100% or more, 200% or more, or 300% or more than a microorganism in which the mutation of the present invention is not introduced (parent strain and/or host microorganism).

The microorganism (or recombinant cell) can additionally comprise mutation so that 3-HP production is increased, and the location of the mutation and/or the type of gene and/or protein to be mutated can be included without limitation as long as it increases 3-HP production. The microorganism can be used without limitation as long as it is a transformable cell.

Herein, in order to distinguish a microorganism before the mutation according to one example is introduced from a microorganism in which the mutation according to one example is introduced so that the 3-HP production capability is increased or the 3-HP production capability is given, the microorganism before the mutation according to one example is introduced can be referred to as a host microorganism (or parent strain).

Also provided is a method for increasing 3-HP production capability of the microorganism or a method for giving 3-HP production capability to the microorganism, comprising introducing (for example, transforming) the aforementioned mutation into a microorganism.

### Production of 3-hydroxypropionic acid (3-HP)

Also provided is a composition for producing 3-hydroxypropionic acid from glucose, comprising a microorganism having production capability of 3-hydroxypropionic acid from the aforementioned glucose.

Also provided is a use for producing 3-hydroxypropionic acid from glucose of a microorganism having production capability of 3-hydroxypropionic acid from the aforementioned glucose.

Also provided is a method for production of 3-hydroxypropionic acid, comprising culturing a microorganism having production capability of 3-hydroxypropionic acid from the aforementioned glucose in a medium containing glucose. The culturing can be performed by adjusting pH 6.5 to 7.0 using a basic solution. In addition, the culturing can comprise adding an inducer which promotes glycerol production (for example, inducer for an inducible promoter) to promote glycerol production.

More specifically, the culturing can comprise adding an inducer for an inducible promoter, and in one example, by addition of the inducer, glycerol production can be promoted, and for example, by addition of the inducer, glycerol production can be promoted as GPD and/or GPP expression is induced. The inducible promoter can be BAD, LTTR, and/or TAC, and when the inducible promoter is BAD, the inducer can be L-arabinose, and when the inducible promoter is LTTR, the inducer can be 3-HP (3-hydroxypropionic acid), and when the inducible promoter is TAC, the inducer is IPTG, and the inducible promoter is as described above.

The microorganism in which the method for production according to one example can be applied can comprise an inducible promoter and a gene encoding glycerol-3-phosphate dehydrogenase (GPD) and a gene encoding glycerol-3-phosphate phosphatase (GPP) which are operably linked thereto, and specifically, it can comprise the aforementioned microorganism.

In one example, the basic solution can be magnesium hydroxide (Mg(OH)₂) solution. In one example, the culturing can be culturing using magnesium hydroxide (Mg(OH)₂) solution under a condition in which pH is adjusted to 6.5 to 7.0, 6.75 to 7.0, 6.9 to 7.0, 6.5 to 6.95, 6.75 to 6.95, or 6.9 to 6.95. In one example, the concentration of the magnesium hydroxide solution can be 10 to 500, 50 to 300, 100 to 200, or 150 g/L.

According to one example, when the pH is adjusted in the above range using magnesium hydroxide, compared to when the pH is adjusted in the above range using other aqueous basic solution (for example, ammonium hydroxide (NH₄OH) solution), the 3-HP production and/or 3HP production yield compared to the input glucose can be increased by about 5% or more, about 10% or more, about 15% or more, about 20% or more, about 30% or more, about 50% or more, about 60% or more, about 70% or more, about 80% or more, about 90% or more, about 100% or more, 200% or more, or 300% or more, but is not limited thereto.

In one example, the medium containing glucose can comprise glucose at a concentration of 5 to 50gL, 10 to 30 g/L, 15 to 25g/L, 10 to 20g/L, 20 to 30g/L, or 20g/L.

The inducer can be added in a culture medium before the microorganism consumes all of the glucose. For example, the production method according to one example can perform culturing by adding the inducer in a culture medium at the beginning of culturing.

In one specific example, the culturing can further comprise adding 3-hydroxypropionic acid to the culture medium before the microorganism consumes all of the glucose. More specifically, in the culturing, before the microorganism consumes all of the glucose, 3-HP is added to the culture medium to induce transcription of GPD and GPP genes, so that the glucose included in the culture medium is used for 3-HP production rather than used for cell growth. By the method according to one example, compared to the conventional method, the 3-HP production compared to the inserted (added) glucose can be increased.

The timing of adding 3-HP in the culturing can be confirmed by measuring whether the microorganism consumes glucose in the culturing, and for example, the amount of consuming glucose by the microorganism can be measured using an oxygen demand. In one example, before the microorganism consumes all the glucose can mean the beginning of culturing (0 hour) to 12 hours, 0 hour to 11 hours and 30 minutes, 0 hour to 11 hours, 0 hour to 10 hours and 30 minutes, or 0 hour to 10 hours.

In one example, before the microorganism consumes all the glucose can mean that the concentration of the glucose in the culture medium is 5%(w/w) or more, 10%(w/w) or more, 15%(w/w) or more, or 20%(w/w) or more, based on the glucose added at the beginning of culturing.

In one example, as the inducer, 3-HP can be added at a concentration of 0.1 to 10g/L, 0.1 to 5 g/L, 0.1 to 3 g/L, 0.5 to 3 g/L, or 0.5 or 1 g/L.

The method for production according to one example can add coenzyme B12 in the culturing, when using a strain not producing coenzyme B12, such as E. coli, and the B12 can be added after the microorganism consumes all the glucose added in the medium. In one example, the B12 can be added when the cell growth of the microorganism has an OD 550 to 650, or an OD 600 value of 5 to 30, 5 to 25, 5 to 20, 5 to 15, 10 to 30, 10 to 25, 10 to 20, 10 to 15, 15 to 30, 15 to 25, or 15 to 20. In another example, the B12 can be added in 5 hours to 30 hours, 10 hours to 30 hours, 10 hours and 30 minutes to 30 hours, 11 hours to 30 hours, 12 hours to 30 hours, 14 hours to 30 hours, 15 to 30 hours, 5 hours to 25 hours, 10 hours to 25 hours, 10 hours and 30 minutes to 25 hours, 11 hours to 25 hours, 12 hours to 25 hours, 14 hours to 25 hours, 15 to 25 hours, 5 hours to 20 hours, 10 hours to 20 hours, 10 hours and 30 minutes to 20 hours, 11 hours to 20 hours, 12 hours to 20 hours, 14 hours to 20 hours, 15 to 20 hours, 5 hours to 15 hours, 10 hours to 15 hours, 10 hours and 30 minutes to 15 hours, 11 hours to 15 hours, 12 hours to 15 hours, 14 hours to 15 hours, 5 hours to 12 hours, 10 hours to 12 hours, 10 hours and 30 minutes to 12 hours, or 11 hours to 12 hours after initiation of culturing.

In one example, the coenzyme B12 can be added at a concentration of 0.1 to 10µM, 0.1 to 5 µM, 0.1 to 3 µM, 0.5 to 3 µM, or 0.5 or 1µM.

The culturing can be achieved according to appropriate medium and culturing conditions known in the art, and it should meet requirements of a specific strain in an appropriate manner, and it can be suitably modified by those skilled in the art.

The culturing method can include for example, batch culture, continuous culture, fed-batch culture or a combination culture thereof, but is not limited thereto.

According to one example, to the medium, various carbon sources, nitrogen sources and microelement components, and the recombinant cell can be cultured while adjusting temperature and/or pH in a common medium containing appropriate carbon sources, nitrogen sources, amino acids, vitamins and the like. The carbon source which can be used includes saccharide and carbohydrate such as glucose, sucrose, lactose, fructose, maltose, starch and/or cellulose, oil and fat such as soybean oil, sunflower oil, castor oil and/or coconut oil, fatty acid such as palmitic acid, stearic acid and/or linoleic acid, alcohol such as glycerol and/or ethanol, and organic acid such as acetic acid. These substances can be used individually or as a mixture, but are not limited thereto. The nitrogen source which can be used can include peptone, yeast extract, meat juice, malt extract, corn steep liquid, soybean flower and urea or inorganic compounds, for example, ammonium sulfate, ammonium chloride, ammonium phosphate, ammonium carbonate and ammonium nitrate. The nitrogen source can be also used individually or as a mixture, but is not limited thereto. The source of phosphorus which can be used can include potassium dihydrogen phosphate or dipotassium hydrogen phosphate, or corresponding sodium-containing salts, but is not limited thereto. In addition, the medium can contain metal salts such as magnesium sulfate or iron sulfate required for growth, but is not limited thereto. In addition, essential growth substances such as amino acids and vitamins can be included. Moreover, appropriate precursors can be used for the medium. The medium or individual components can be added batchwise or continuously by an appropriate method to the culture solution during the culture process, but is not limited thereto.

According to one example, the pH of the culture solution can be adjusted by adding a compound such as ammonium hydroxide, potassium hydroxide, ammonia, phosphoric acid and sulfuric acid to the microbial culture solution during culturing. In addition, during culturing, bubble formation can be inhibited using an antifoaming agent such as fatty acid polyglycol ester. Additionally, in order to maintain the aerobic state of the culture solution, oxygen or oxygen-containing gas (e.g., air) can be injected into the culture solution. The temperature of the culture solution can be 20°C to 45°C, 25°C to 40°C, or 30°C to 37°C. The culture period can be continued until a useful substance (for example, 3-HP) is obtained in a desired yield, and for example, it can be 10 to 160 hours.

In one example, the production method can further comprise separating, recovering and/or purifying 3-hydroxypropionic acid from the cultured microorganism and culture medium.

The separating and/or recovering 3-HP from the cultured microorganism (or recombinant cell) and/or culture medium can collect a target substance (for example, 3-HP) from the medium, culture solution or microorganism using an appropriate method known in the art according to the culturing method. For example, a method of centrifugation, filtering, extracting, spraying, drying, evaporation, precipitation, crystallization, electrophoresis, fractional dissolution (for example, ammonium sulfate precipitation) and/or chromatography (for example, ion exchange, affinity, hydrophobic, liquid and size exclusion), and the like can be used, but is not limited thereto. The culture medium can mean a medium culturing a microorganism (or recombinant cell).

In one example, the method for producing 3-HP can additionally comprise purifying 3-HP before, simultaneously with, or after the separating and/or recovering.

### Culture comprising 3-hydroxypropionic acid (3-HP)

Also provided is a culture of culturing the aforementioned microorganism in a medium comprising glucose. The culture can comprise 3-HP at a high concentration. The culture can comprise by-products at a low level. The culture can comprise or not comprise (cell-free) the microorganism.

More specifically, the culture can comprise 3-HP at a concentration of 60g/L or more, 65g/L or more, 70g/L or more, or 75g/L or more, for example, 60g/L to 200g/L, 60g/L to 150g/L, 60g/L to 100g/L, 65g/L to 200g/L, 65g/L to 150g/L, 65g/L to 100g/L, 70g/L to 200g/L, 70g/L to 150g/L, 70g/L to 100g/L, 75g/L to 200g/L, 75g/L to 150g/L, or 75g/L to 100g/L.

In addition, the culture can comprise one or more selected from the group consisting of glycerol, acetate and PDO (1,3-propanediol), for example, glycerol at a concentration of 20g/L or less, 15g/L or less, 10g/L or less, 5g/L or less, 4g/L or less, 3g/L or less, 2g/L or less, or 1.5g/L or less (the lower limit can be 0 (not including by-products), 0.001g/L, 0.01g/L, or 0.1g/L).

### [ADVANTAGEOUS EFFECTS]

The microorganism according to one example can have an increased 3HP production capability (3HP production) by comprising a mutation that allows glucose introduced into cells to be used for 3HP production rather than cell growth, and the method according to one example can increase the 3HP production yield, by adjusting the addition timing of an inducer and/or adjusting the type of aqueous base solution used for pH adjustment.

### [BRIEF DESCRIPITON OF THE DRAWINGS]

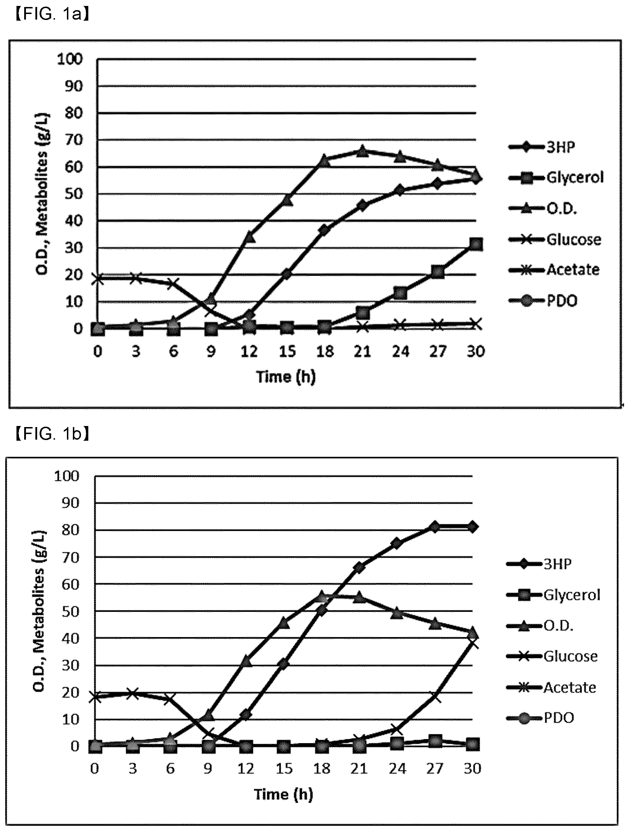

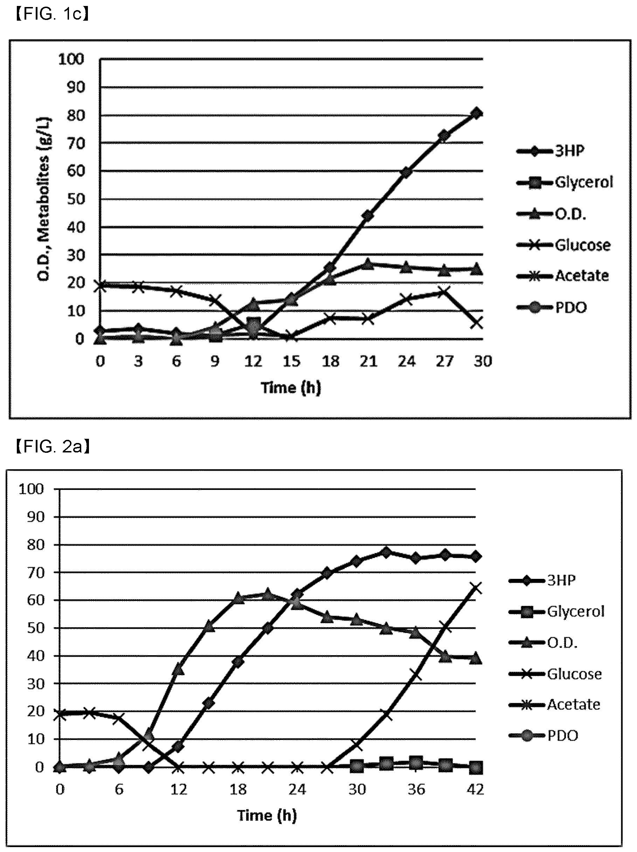

FIG. 1a to FIG. 1c show the results of measuring growth (OD) and the amount of substances produced by the mutant strain of Experimental group 1 (FIG. 1b) and Experimental group 2 (FIG. 1c) of Table 8, which are mutant strains according to Comparative example of Table 8 (FIG. 1a), and one example over time.

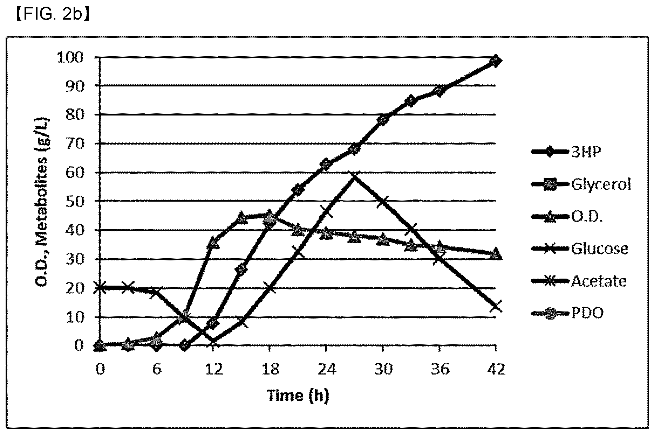

FIG. 2a and FIG. 2b are results of measuring growth (OD) and the amount of substances produced by the mutant strain according to one example over time, and specifically, FIG. 2a shows the result in the case of adjusting pH using NH₄(OH) in the culturing (Experimental group 3 of Table 9), and FIG. 2b shows the result of adjusting pH using Mg(OH)₂ in the culturing (Experimental group 4 of Table 9).

### [MODE FOR INVENTION]

The present invention will be described in more detail by the following examples, but the scope is not intended to be limited by the following examples.

### Example 1. Mutant strain construction

### Example 1-1. pRSF-pLTTR-GPD-GPP

GPD1 and GPP2 genes derived from Saccharomyces cerevisiae were under PCR (preliminary denaturation at 95°C for 2 minutes; repeating denaturation at 95°C for 1 minute, annealing at 55°C for 30 seconds and elongation at 72°C for 2 minutes 28 times; final elongation at 72°C for 5 minutes; stored maintained at 4 degrees) using the primer pair of SEQ ID NOs: 1 and 2 or SEQ ID NOs: 3 and 4 of Table 2 below, respectively, and treated with BamHI/Sacl and Kpnl/Xhol restriction enzymes, respectively, and cloned into pRSFDuet-1 vector (Invitrogen) comprising LTTR promoter, to produce a vector comprising pRSF-pLTTR-GPD-GPP (hereinafter, "pRSF-pLTTR-GPD-GPP vector"; the vector names described herein indicate the gene/promoter comprised in the vector and their position (order) from the 5' end). The sequence of the LTTR promoter used is described in Table 3 below.

**[Table 2]**

| Name | Sequence (5'->3') | SEQ ID NO: |
|---|---|---|
| GPD1-F | CATCATCATGGATCCA TAGCAGTAAGAAAGGAGCATCCATCatgagcgctgcggctg | 1 |
| GPD1-R | CATCATCATGAGCTCtcaatcttcatgaaggtctaattcttcaatcatg | 2 |
| GPP2-F | CATCATCATGGTACCACAGTTTTAGTAAAGGAGCATCAAAA atgggactcactacgaaaccg | 3 |
| GPP2-R | catcatcatCTCGAGttaccatttcagcagatcgtctttgg | 4 |

**[Table 3]**

| Name | Sequence (5'->3') | SEQ ID NO: |
|---|---|---|
| LTTR | GTCAGCCTCAGCGCACCTCGAATGTGCAAAAACGCAGACCATA CTTGCACATCACCGCATTGAGTACATCAAAAATGCACTGTTAGG ATCGATCCAGACAACAAAAAAGCCACA | 5 |

### Example 1-2. pCDF_J23101_dhaB_gdrAB_J23100_aldH_btuR construction

A gene encoding glycerol dehydratase (dhaB), a gene encoding aldehyde dehydrogenase (aldH) and a gene encoding glycerol dehydratase reactivase (gdrAB) were cloned into a plasmid pCDF to produce 'pCDF_J23101_dhaB_gdrAB_J23100_aldH', and a gene encoding adenosyltransferase (btuR) was cloned thereto to produce 'pCDF_J23101_dhaB_gdrAB_J23100_aldH_btuR '.

Specifically, the promoter part of the pCDFDuet-1 vector (Novagen, USA) was substituted with J23101 and J23100 promoters. The dhaB (U30903.1; about 2.7 kb; dhaB1, dhaB2, dhaB3) and gdrAB gene (about 2.2 kb; gdrA, gdrB) were amplified using the primer pair of SEQ ID NOs: 6 and 7 or SEQ ID NOs: 8 and 9 of Table 4 below in the chromosome of Klebsiella pneumoniae (ATCC 25955) using the condition provided by NEB:

**[Table 4]**

| Name | Sequence (5'->3') | SEQ ID NO: |
|---|---|---|
| dhaB-gdrA-F | GAATTCATGAAAAGATCAAAACGATTTGCAGTCCT | SEQ ID NO: 6 |
| dhaB-gdrA-R | AAGCTTGATCTCCCACTGACCAAAGCTGG | SEQ ID NO: 7 |
| gdrB-F | AAGCTTAGAGGGGGCCGTCATGTCGCTTTCACCGCCAG | SEQ ID NO: 8 |
| gdrB-R | CTTAAGTCAGTTTCTCTCACTTAACGGC | SEQ ID NO: 9 |

The dhaB123 and gdrA genes were positioned side by side on the chromosome of Klebsiella pneumonia, and therefore, they were amplified together, and gdrB was positioned on the opposite direction to dhaB123, gdrA, and therefore, gdrB was amplified separately, and then, NEB Q5 DNA polymerase was used, and for the condition, the condition provided by NEB was used. After that, the dhaB123, gdrA genes were cloned using restriction enzymes EcoRI and Hindlll, and the gdrB gene was cloned using restriction enzymes Hindlll and Aflll under the J23101 promoter (3' end).

aldH was cloned under the J23100 promoter (3' end), and using primers below, it was amplified from E. coli K12 MG1655 chromosome, and then the primers of Table 5 and NEB Q5 DNA polymerase were used, and for the condition, the condition provided by NEB was used. aldH was cloned on the bottom of the J23100 promoter using restriction enzymes Kpn I and Nde I . btuR was cloned below aldH, and it was amplified from E. coli K12 MG1655 Chromosome using promoters below, and then, the primers of Table 5 and NEB Q5 DNA polymerase were used, and for the condition, the condition provided by NEB was used. Cloning of btuR was performed under aldH using In-Fusion Kit of Takara. Through the above cloning, 'pCDF_J23101_dhaB_gdrAB_J23100_aldH_btuR ' vector was produced.

**[Table 5]**

| Name | Sequence (5'->3') | SEQ ID NO: |
|---|---|---|
| aldH-F | ggtaccatgaattttc atcatctggc | SEQ ID NO: 10 |
| aldH-R | catatgtcaggcctccaggcttat | SEQ ID NO: 11 |
| btuR-F | tggaggcctgacataatgagtgatgaacgctaccaacag | SEQ ID NO: 12 |
| btuR-R | ttgagatctgccatattaataatcgatccctatctgcgc | SEQ ID NO: 13 |

As a control group, a microorganism in which a plasmid pCDF comprising a gene encoding glycerol dehydratase (dhaB), a gene encoding aldehyde dehydrogenase (aldH) and a gene encoding glycerol dehydratase reactivase (gdrAB), in which btuR gene was not cloned, was introduced by the same method was used.

The prepared vector was introduced into E. coli W3110 (KCCM 40219) by electroporation using an electroporation device (Bio-Rad, Gene Pulser Xcell), and thereby, the 3-HP producing strain according to one example of the present application and the control strain were produced, respectively. The strains were cultured in a shaking incubator under the condition of 37°C and 250 rpm after inoculating a single colony of the corresponding strain to a LB medium comprising 25mg/L streptomycin (containing yeast extract 5 g/L, tryptone 10g/L, and NaCl 10g/L).

### Example 1-3. Deletion of ptsG gene and overexpression of galP and glk genes

ptsG gene was deleted using Cas9 system. Specifically, an expression vector comprising Cas9 gene (Quick&easy E. coli gene deletion kit (K006) of Gene Bridges) was transformed into DKAPG strain (strain which yqhD, glpK, IdhA, ack-pta and gldA genes were deleted from W3110 strain), and a guide RNA vector targeting the ptsG gene part and repair DNA (Quick&easy E. coli gene deletion kit (K006) of Gene Bridges) were transformed simultaneously thereto to obtain DKALGP::PK. The ptsG front part (1000 bp at the 5' end) and back part (1000 bp at the 3' end) were amplified from E. coli K12 MG1655 chromosome using primers of SEQ ID NOs: 14 and 15 and SEQ ID NOs: 18 and 19.

For overexpression of galP and glk genes, galP and glk genes were amplified using primers of SEQ ID NOs: 16 and 17 from the pRSF-J23106-galP-glk-LTTR-GPD-GPP vector. The amplified three fragments (ptsG front part, ptsG back part, and galP and glk genes) were used as Repair-DNA by Overlap-PCR using SEQ ID NOs: 14 and 19. The sequences of the primers used in the present example are described in Table 6 below. In case of the pRSF-J23106-galP-glk-LTTR-GPD-GPP vector, galP and glk genes were amplified by PCR using the genomic DNA of W3110 strain and primers of SEQ ID NOs: 20 and 21 and the primer pair of SEQ ID Nos: 22 and 23, respectively, and amplified by Overlap-PCR using a template in which the amplified two PCR products were mixed and the primer pair of SEQ ID NOs: 20 and 23, and the final galP-glk fusion gene constructed by amplifying by Overlap-PCR was constructed through pRSF-pLTTR-GPD-GPP vector linearized with Kpnl/Ndel restriction enzymes and In-fusion reaction (50°C 30 minutes).

**[Table 6]**

| Name | Sequence (5'->3') | SEQ ID NO: |
|---|---|---|
| ptsG-1000-F | ACGCGATGTGAAATTTTGTC | SEQ ID NO: 14 |
| ptsG-1000-R | CACTATACCTAGGACTGAGCTAGCCGTAAAAATTG AGAGTGCTCCTGAGTATG | SEQ ID NO: 15 |
| J23-over-F | TTTACGGCTAGCTCAGTCC | SEQ ID NO: 16 |
| glk-over-R | ttacagaatgtgacctaaggtctg | SEQ ID NO: 17 |
| ptsG+1000-F | catttacgccagaccttaggtcacattctgtaaTCCGTAAGACGTT GGGGAG | SEQ ID NO: 18 |
| ptsG+1000-R | CCAACATCTTCCCGGATG | SEQ ID NO: 19 |
| galP_infu_F | tagtgctagcGGTACatgcctgacgctaaaaaacaggggc | SEQ ID NO: 20 |
| galP_infu_R | GTCAAAATAATTAATCGTGAGCGCCTATTTC | SEQ ID NO: 21 |
| glk_infu_F | TCACGATTAATTATTTTGACTTTAGCGGAGCAG | SEQ ID NO: 22 |
| glk_infu_R | TCCAATTGAGATCTGCCATATTACAGAATGTGACCT AAGGTCTGG | SEQ ID NO: 23 |

### Example 1-4. Mutant strain construction

Using Quick&easy e. coli gene deletion kit of Gene Bridges, according to the manual of the manufacturer, a strain W3110 which yqhD, glpK, IdhA, ack-pta and gldA genes were deleted from W3110 (purchased from KCTC) strain (ΔyqhD, ΔglpK, ΔldhA, Δack-pta and ΔgldA) (named DKALG strain) was constructed, and as the method of Example 1-3, ptsG gene was deleted from the DKALG strain using Cas9 system, and at the ptsG gene position, galP and glk genes were inserted and thereby, a strain in which galP and glk genes were overexpressed (named DKALGP::PK strain) was constructed.

The pRSF-pLTTR-GPD-GPP vector and pCDF J23101 dhaB_gdrAB J23100 aldH_btuR vector produced in Example 1-1 and Example 1-2 were introduced by electroporation using an electroporation device (Bio-Rad, Gene Pulser Xcell) into the DKALGP::PK strain and transformed, to construct a strain comprising a gene encoding glycerol dehydratase (dhaB), a gene encoding aldehyde dehydrogenase (aldH), a gene encoding glycerol dehydratase reactivase (gdrAB) and a gene encoding adenosyltransferase.

As a control group, by introducing the pRSF-pLTTR-GPD-GPP vector and pCDF J23101 dhaB_gdrAB J23100 aldH vector constructed in Example 1-1 and Example 1-2 into the DKALGP::PK strain by the same method, a microorganism, in which a plasmid pCDF in which a gene encoding glycerol dehydratase (dhaB), a gene encoding aldehyde dehydrogenase (aldH), and a gene encoding glycerol dehydratase reactivase (gdrAB) were comprised and btuR gene was not cloned, was used.

Specifically, the DKALGP::PK strain was plated on an LB plate (comprising 10g tryptone, 5g yeast extract, 10g NaCl, 15g agar per 1 L), respectively, and cultured at 37°C overnight. The cultured single colony was inoculated in a 3 to 5mL LB liquid medium (same medium composition as above, not comprising agar) and cultured at 37°C overnight with stirring. Next morning, the culture solution was diluted in the LB liquid medium of 0.05 liters by 1:100 and cultured for 3-6 hours until an OD of ~0.5. After cooling on ice for 10 to 15 minutes, cells were collected by centrifuging at 4°C at 4000rpm for 10 minutes. The cells were resuspended in sterile DI water 1 volume cooled on ice and washed, and then centrifuged at 4°C at 4000rpm for 10 minutes to collect the cells. The collected cells were resuspended in DI water 0.5 volume and washed and then centrifuged at 4°C at 4000rpm for 10 minutes to collect the cells again. The collected cells were resuspended in sterile 10% glycerol 1mL cooled on ice, and cell aliquots were put in an individual pre-chilled microfuge tube (90µl per transformation in a cuvette for gap electroporation of 0.1cm). Salt-free DNA (vector) 1-5µL was added by pipetting, and then the mixture was moved into a pre-chilled electroporation cuvette. Immediately after electroporation at 1.8kV and pulse, 1mL of the LB liquid medium at a room temperature was added to the cells and cultured at 37°C for 1 hour. The culture solution was plated on an LB plate comprising kanamycin and streptomycin and excess liquid was allowed to dry/absorb into the plate. The plate was inverted and cultured at 37°C.

A strain into which the pRSF-pLTTR-GPD-GPP vector (GG) and pCDF J23101 dhaB_gdrAB J23100 aldH vector (DGA) were introduced into the DKALGP::PK strain (hereinafter, named DKALGP::PK + GG-DGA strain) and a strain into which the pRSF-pLTTR-GPD-GPP vector (GG) and pCDF J23101 dhaB_gdrAB J23100 aldH_btuR vector (DGAB) were introduced into the DKALGP::PK strain (hereinafter, named DKALGP::PK + GG-DGAB strain) were constructed.

### Example 2. 3-HP production measurement

### Example 2-1. Mutation introduction and measurement of 3-HP production according to time of addition of inducer

A single colony of the strain prepared in Example 1 was inoculated in an LB medium and was under seed culture under the condition of 37°C for 18 hours.

Then, in a 5L fermenter, seed culture 150mL was inoculated into a modified MR medium of 2L comprising 20 g/L glucose and 25mg/L streptomycin and 50 mg/L kanamycin (containing MgSO₄ • 7H₂O 0.8 g/L, (NH₄)₂HPO₄ 4 g/L, KH₂PO₄ 6.67 g/L and citrate 0.8 g/L) in a flask, and in a fermenter of 35°C, 500 rpm, it was cultured under the condition of maintaining pH as 6.95 using NH₄(OH) or Mg(OH)₂.

At the time of Condition 1 or Condition 2 below, by adding 100 g/L 3HP 10mL, 5mM B12 10 mL, 3HP production was induced, and after exhausting glucose comprised in the medium, Feeding Solution (comprising glucose 700g/L, MgSO₄ 15g/L, Trace metal 10mL/1L, 10x MR salt 100mL/1L, 25mg/L streptomycin, and 50mg/L kanamycin) was added at a rate of 40.5 mL/hr. Under Condition 1 and Condition 2, the time points at which glucose was all consumed were 10.5 H (hour) and 11.5H (hour) after the start of culture, respectively.
- **Condition 1**: at 10.5H when glucose was all consumed, adding 100 g/L 3HP 10mL, 5mM B12 10mL
- **Condition 2**: at the beginning of culture (0 hour), adding 100 g/L 3HP 10mL, and at 11.5H when glucose was all consumed, adding 5mM B12 10mL

After centrifuging (12,300rpm, 1 minute) the culture solution 1mL cultured under each condition for 24 hours, the supernatant was prepared using a filter in a 0.45µm size. Then, using HPLC (High-Performance Liquid Chromatography), the 3-HP, glycerol production and production of by-products (acetate and PDO) in the culture solution were analyzed, and the result was shown in Table 8 and FIG. 1a (Comparative example of Table 8) and FIG. 1b (Example 1 of Table 8). Specifically, HPLC was performed under the condition of Table 7 below.

In addition, the growth of the strain under each culture condition was measured by measuring OD600 (optical density (O.D.) of the strain culture solution at 600nm) using a UV Spectrometer, and the result was shown in Table 8 and FIG. 1a (Comparative example of Table 8), FIG. 1b (Experimental group 1 of Table 8) and FIG. 1c (Experimental group 2 of Table 8).

**[Table 7]**

| HPLC Model | Agilent Technologies 1200 Series |
|---|---|
| Column | Bio-Rad Aminex HPX-87H Ion Exclusion Column300 |
| | mm × 7.8 mm |
| Detector | Refractive Index (Rl) / Ultraviolet (UV) Detector |
| Mobile Phase | 0.5 mM H₂SO₄ |
| Flow rate | 0.4 mL/min |
| Run time | 35 min |
| Column temperature | 35°C |
| Detector temperature | 35°C |
| Injection volume | 10µ*ℓ* |

**[Table 8]**

| | Strain | Culture condition | | O.D. | Glycerol (g/L) | 3HP yield (g-3HP/g-glucose) | 3HP (g/L) |
|---|---|---|---|---|---|---|---|
| | | pH adjustment | 3HP addition time | | | | |
| Comparative example | DKALGP::PK + GG-DGA | NH₄(OH) | Condition 1 | 64.0 | 13.4 | 0.399 | 51.4 |
| Experimental group 1 | DKALGP::PK + GG-DGAB | NH₄(OH) | Condition 1 | 49.6 | 1.01 | 0.545 | 75.2 |
| Experimental group 2 | DKALGP::PK + GG-DGAB | Mg(OH)₂ | Condition 2 | 25.6 | 0 | 0.761 | 59.5 |

As shown in Table 8, and FIG. 1a to FIG. 1c, in case of the strain in which the btuR gene was overexpressed (DKALGP::PK + GG-DGAB; Experimental group 1 and Experimental group 2), compared to Comparative example (DKALGP::PK + GG-DGA), the accumulated amount of glycerol and cell growth were reduced, and the 3HP production and 3HP yield (ratio of 3HP production to added glucose; g/g) were significantly increased. In addition, in Experimental group 1 (FIG. 1b) and Experimental group 2 (FIG. 1c), production of by-products was hardly observed. In addition, as shown in Table 8, it could be confirmed that the accumulated amount of glycerol and cell growth were reduced and the 3HP yield (ratio of 3HP production to added glucose) was significantly increased, in case of Experimental group 2, compared to Comparative example and Experimental group 1.

Accordingly, it could be confirmed that the mutant strain (btuR gene overexpression) according to one example and/or method for producing 3HP using the mutant strain significantly increased the 3HP production yield from glucose, and glucose could be utilized for 3HP production rather than cell growth.

### Example 2-2. Measurement of 3HP production according to kind of aqueous basic solution

Similar to the method of Example 2-1, the strain in which the btuR gene was overexpressed constructed in Example 1 (DKALGP::PK + GG-DGAB) was cultured under Condition 1, using NH₄(OH) or Mg(OH)₂, under a condition of maintaining pH as 6.95 for 42 hours, and the production of 3-HP, glycerol and by-products (acetate and PDO) in the culture solution and the growth of the strain were measured, and the result was shown in Table 9 and FIG. 2a (Experimental group 3 of Table 9) and FIG. 2b (Experimental group 4 of Table 9) below.
- **Condition 1**: at 10.5H when glucose was all consumed, adding 100 g/L 3HP 10mL, 5mM B12 10mL

**[Table 9]**

| | Strain | Culture condition | | 3HP (g/L) |
|---|---|---|---|---|
| | | pH adjustment | 3HP addition time | |
| Experimental group 3 | DKALGP::PK + GG-DGAB | NH₄(OH) | Condition 1 | 75.6 |
| Experimental group 4 | | Mg(OH)₂ | | 98.7 |

As shown in Table 9, FIG. 2a and FIG. 2b above, it could be confirmed that the 3HP production was significantly increased in Experimental group 4 using Mg(OH)₂ compared to Experimental group 3 using NH₄(OH) to adjust pH in the culturing.

## Claims

1. A microorganism of the genus Escherichia, in which:
(1) an activity of adenosyltransferase is enhanced, and
(2) an activity of glycerol-3-phosphate dehydrogenase (GPD), glycerol-3-phosphate phosphatase (GPP), or both GDP and GPP are enhanced.

2. The microorganism according to claim 1, further having one or more characteristics selected from the following:
(3) enhancement of activity of one or more enzymes selected from the group consisting of glycerol dehydratase, glycerol dehydratase reactivase, and aldehyde dehydrogenase;
(4) enhancement of activity of galactose permease, glucokinase, or both of them; and
(5) weakening or inactivation of activity of a glucose-specific transporter of the phosphotransferase system.

3. The microorganism according to claim 1, wherein the activity of one or more enzymes selected from the group consisting of (1) to (6) below is weakened or inactivated:
(1) 1,3-propanediol dehydrogenase;
(2) glycerol kinase;
(3) acetate kinase;
(4) phosphotransacetylase;
(5) lactate dehydrogenase; and
(6) glycerol dehydrogenase.

4. The microorganism according to claim 2, having all the following characteristics:
(1) enhancement of activity of adenosyltransferase;
(2) enhancement of activity of glycerol-3-phosphate dehydrogenase (GPD) and glycerol-3-phosphate phosphatase (GPP);
(3) enhancement of activity of glycerol dehydratase, glycerol dehydratase reactivase, and aldehyde dehydrogenase;
(4) enhancement of activity of galactose permease and glucokinase; and
(5) weakening or inactivation of activity of a glucose-specific transporter of the phosphotransferase system.

5. The microorganism according to claim 1, wherein the enhancement of activity of adenosyltransferase is due to increased expression of a btuR gene.

6. The microorganism according to claim 1, wherein the enhancement of activity of glycerol-3-phosphate dehydrogenase and glycerol-3-phosphate phosphatase is due to increased expression of a gene encoding glycerol-3-phosphate dehydrogenase and a gene encoding glycerol-3-phosphate phosphatase.

7. The microorganism according to claim 1, wherein the microorganism is an E. *coli.*

8. The microorganism according to claim 1, wherein the microorganism exhibits inhibited cell growth.

9. The microorganism according to any one claim of claim 1 to claim 8, having production capability of 3-hydroxypropionic acid from glucose.

10. A composition for producing 3-hydroxypropionic acid from glucose, comprising the microorganism according to any one claim of claim 1 to claim 8.

11. A method for production of 3-hydroxypropionic acid from glucose, comprising culturing the microorganism according to any one claim of claim 1 to claim 8 in a medium containing glucose.

12. The method of claim 11, wherein the culturing is performed under a controlled pH condition by adjusting pH to 6.5 to 7.0 using a basic solution.

13. The method of claim 12, wherein the basic solution is a magnesium hydroxide solution.

14. The method of claim 11, wherein the culturing comprises adding an inducer for promoting glycerol production into a culture medium before the microorganism consumes all of the glucose.

15. The method of claim 14, wherein the inducer is added into a culture medium from the beginning of the culturing.

16. The method of claim 11, wherein the inducer is 3-hydroxypropionic acid.

17. A culture obtained by culturing the microorganism of any one claim of claim 1 to claim 8 in a medium comprising glucose.

18. The culture according to claim 17, comprising 3-hydroxypropionic acid at a concentration of 60g/L to 200g/L and comprising glycerol at a concentration of 10g/L or less.
